Europäisches Patentamt

European Patent Office

Office européen des brevets

(11) Publication number: **0 168 201**
**B1**

(12) **EUROPEAN PATENT SPECIFICATION**

(45) Date of publication of patent specification: **22.06.88**

(51) Int. Cl.⁴: **A 61 F 9/00**

(21) Application number: **85304597.9**

(22) Date of filing: **27.06.85**

(54) Aqueous humour drainage device.

(30) Priority: 28.06.84 GB 8416544
15.10.84 GB 8425946
30.11.84 GB 8430221
26.02.85 GB 8504905

(73) Proprietor: **Joseph, Neil Howard**
**54 Hereford Road**
**London W2 (GB)**

(43) Date of publication of application:
**15.01.86 Bulletin 86/03**

(72) Inventor: **Joseph, Neil Howard**
**54 Hereford Road**
**London W2 (GB)**

(45) Publication of the grant of the patent:
**22.06.88 Bulletin 88/25**

(74) Representative: **Pratt, David Martin et al**
**Brookes & Martin High Holborn House 52/54**
**High Holborn**
**London. WC1V 6SE (GB)**

(84) Designated Contracting States:
**AT BE CH DE FR IT LI LU NL SE**

(56) References cited:
EP-A-0 102 747
FR-A-2 233 028
GB-A-2 101 891
SU-A- 906 561
SU-A- 950 378
US-A-3 915 172
US-A-4 030 480
CANADIAN JOURNAL OF OPHTHALMOLOGY,
vol. 9, October 1974, pages 463-467; G.A.
PEYMAN et al.: "An experimental "aqueous
shunt" for the regulation of intraocular
pressure"

(56) References cited:
MEDICAL & BIOLOGICAL ENGINEERING &
COMPUTING, vol. 15, no. 5, September 1977,
pages 564-572; M.S. Engleman et al.:
"Computer localisation of detatched retinal
tears for a scleral buckling procedure"

ARCHIVES OF OPHTHALMOLOGY, vol. 83, May
1970, pages 613-618; G.A. CALABRIA et al.:
"Sutureless scleral buckling"
SURGERY, vol. 52, no. 2, August 1962, pages
385-390; S. ORE et al.: "Preparation of surgical
implants from silicone rubber by means of a
postforming technique"

EP 0 168 201 B1

## Description

This invention relates to a device for draining aqueous humour from a human eye for the relief of glaucoma, according to the first part of Claim 1.

Glaucoma is an eye condition in which, from various causes, the intra-ocular pressure (that is to say the pressure of the aqueous humour in the eye) rises, tending to make the eyeball hard and, in high-tension glaucoma, adversely affecting vision and even causing partial or total loss of sight.

A known method of treating glaucoma is filtration surgery, either with or without implanted drainage devices. In a filtering operation which does not involve an implanted drainage device, an opening is cut into the anterior chamber of the eye (the opening being formed under a flag of conjuctiva) to drain aqueous humour to a bleb which forms over the sclera. The aqueous humour in the bleb seeps away over a period to small blood vessels and lymphatic vessels. Filtering operations of this type are reasonably successful. Unfortunately, however, about 20% fail over a period of time following the operation, and about 30% lead to cataract formation. The main reason for these problems is a result of poor control of the filtering opening, and subsequent reduction in the drainage flow of aqueous humour. The resulting ocular hypotony may lead to cataract formation, and does lead to the apposition of tissues and scarring at the site of the sclerostomy, and this gives rise to failure of the operation.

Although various attempts have been made to overcome these problems by the use of implant devices, none of these has proved entirely successful. Implant devices utilise drainage tubes to maintain the integrity of the openings formed in diseased eyes for the flow of aqueous humour. Typically, the drainage tube of such a device leads to a drainage body which is sutured to the sclera. The purpose of the drainage body is to increase the drainage area, and hence to ensure that aqueous humour drains away at a sufficiently high rate. After such a device is surgically implanted, scar tissue forms around the drainage body, the aqueous humour filtering through this scar tissue to the blood and lymphatic vessels. This scar tissue helps to immobilise the device. However, if the scar is too thick, it resists filtration of aqueous humour to the surrounding blood and lymphatic vessels. In this connection, a thick scar is analogous to multple layers of filter paper, in that both require a larger surface area (than a thin scar or a single layer of filter paper) to ensure the same rate of filtration. Thus, when thick scar tissue forms with this type of device, failure follows owing to inadequate surface area of the drainage body. In a normal eye, the pressure of aqueous humour in the anterior chamber is about 14 to 16 mm of mercury. A device may be said to have failed, when the pressure in the anterior chamber is greater than 18 mm of mercury. A medically accepted test for a successful drainage device is one that ensures that the intra-ocular pressure remains below 18 mm of mercury for at least six months following implantation.

A typical known implant device (see GB—A—2 101 891) utilises a thin silicone rubber drainage tube which leads to one or more ridged circular plates. The circular plate(s) form a drainage body, which is sutured to the sclera of the eye. This type of device has a high failure rate, because the surface area of the drainage body is inadequate to ensure sufficient drainage of aqueous humour.

Another known implant device (see EP—A—0 102 747) includes a limp reservoir adapted to be worn against or within the eye wall. A conduit leads from the reservoir into the anterior chamber of the eye, and another conduit joins the reservoir with liquid-receptive tissue. A one-way check valve is provided for preventing back-flow of liquid into the anterior chamber. This device also has a high failure rate, because the surface area of the implant is inadequate to ensure sufficient drainage of aqueous humour. Moreover, the conduit joining the reservoir to the liquid receptive tissue is prone to being completely covered by scar tissue, thus preventing adequate filtration of aqueous humour.

Yet another known implant device see "Canadian Journal of Ophthalmology"—Vol. 9, October 1974, pages 463—467) is constituted by an aqueous shunt. This device has a high failure rate because the drained aqueous humour does not lead to a scar-maintaining drainage body. Consequently, there is insufficient surface area to maintain an absorption scar of adequate absorption ability to deal with the aqueous humour produced by the eye at a physiological pressure gradient. As a result, scar tissue forms around the device, and eventually this prevents filtration of aqueous humour to the surrounding blood and lymphatic vessels, and hence to failure of the device.

The aim of the invention is to provide an aqueous humour drainage device which does not suffer from the disadvantages of known devices, and which can ensure that the intra-ocular pressure remains below 18 mm of mercury for at least six months following implantation for the majority of patients.

The present invention provides a device for draining aqueous humour from an eye, the device comprising a drainage tube for draining aqueous humour from the anterior chamber of an eye, and a drainage body for distributing drained aqueous humour over a relatively large area, the drainage body being sutured, in use, to the sclera of the eye, the drainage tube being firmly fixed to the drainage body and opening directly on to a surface of the drainage body, the device being provided with a pressure gradient limiting valve having a predetermined opening pressure, characterised in that the drainage body is constituted by a band having a width of at least 5 mm and a length which is sufficient for the band to pass over the sclera of the eye in an equatorial position, the band having a surface area which is at least equal to that of a band of width 5 mm and a length sufficient to pass around the sclera of the eye in an equatorial position, and in that the valve is positioned in the region where the tube is firmly fixed to the band.

In a preferred embodiment, the band is long enough to encircle the eye completely at said equatorial position.

The use of a band of such a width and length ensures that there is a sufficiently large filtration area to ensure that the intra-ocular pressure remains below 18 mm of mercury for at least six months following implantation for the majority of patients. Moreover, the fixing of the tube to the band prevents scar tissue forming around the opening (which otherwise could block the flow of aqueous humour to said surface of the band). The provision of the pressure gradient limiting valve ensures that the initial flow of aqueous humour away from the anterior chamber is restricted to a level which permits adequate circulation of aqueous humour over the lens of the eye.

Advantageously, the pressure gradient limiting valve is positioned at that end of the tube which is fixed to the band, the valve constituting the opening through which aqueous humour flows directly to said surface of the band. Preferably, the pressure gradient limiting valve is constituted by at least one slit formed in the wall of the tube.

In a preferred embodiment, the tube is preformed to follow the arc of a circle, the diameter of which is such that, in use, the free end of the tube is positioned within the anterior chamber of the eye closer to the iris than the cornea. Preforming the tube in this way ensures that the free end of the tube can easily be positioned in the anterior chamber well clear of the cornea, and this is advantageous as the tube could damage the cornea if it touches the cornea. For most eyes, the diameter of said circle is preferably about 30 mm.

Preferably, the tube and the band are made of physiologically inert material such as medical grade silicone rubber or a hydrogel (such as hydroxyethylmethacrylate).

For most eyes, the band preferably has a width lying in the range of from 9 to 12 mm, and the pressure gradient limiting valve has an opening pressure of from 4 to 20 mm of mercury. Conveniently, the tube is fused or glued to the band.

In a preferred embodiment, the tube extends beyond the band, the end of the extended portion being closed. In this case, the device may further comprise an additional drainage body and an additional tube, one end of the additional tube being connectible to the extension of the first-mentioned tube, after said closed end thereof has been opened, the other end of the additional tube being fixed to the additional drainage body and opening directly on to a surface of the additional drainage body. Advantageously, an additional pressure gradient limiting valve is provided in the additional tube. The additional drainage body can be implanted (preferably under the scalp) some time after the initial operation, if the intra-ocular pressure rises too high owing to inadequate filtration resulting from excessive scar formation. This can be accomplished by simple extra-ocular surgery. If necessary, further drainage area can subsequently be provided by connecting a further drainage body to the additional drainage body to make a physically continuous body.

Two forms of aqueous humour drainage device, each of which is constructed in accordance with the invention, will now be described in detail, by way of example, with reference to the accompanying drawings, in which:—

Fig. 1 is a schematic cross-section through the first form of device;

Fig. 2 is a schematic representation showing the device of Fig. 1 implanted in an eye;

Figs. 3a to 3d are enlarged schematic views showing alternative valve arrangements that can be incorporated in the device of Fig. 1;

Fig. 4 is a view similar to that of Fig. 2, showing the second form of device implanted in an eye;

Fig. 5 is a view similar to that of Fig. 4, showing a modified form of device having facilities for adding an additional drainage body;

Fig. 6 is an enlarged cross-sectional view of an arrangement for connecting an additional drainage body;

Fig. 7 is a plan view of the second form of device together with an additional drainage body; and

Fig. 8 is a view similar to that of Fig. 7, and shows the addition of a further drainage body.

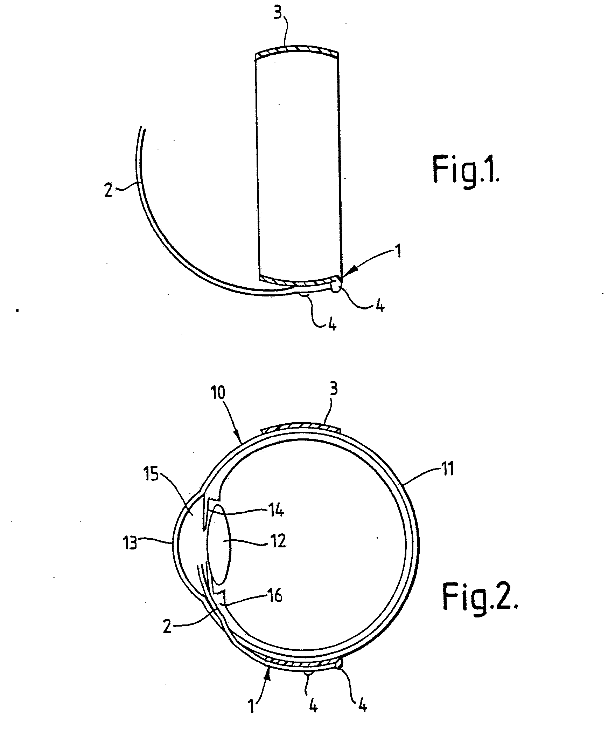

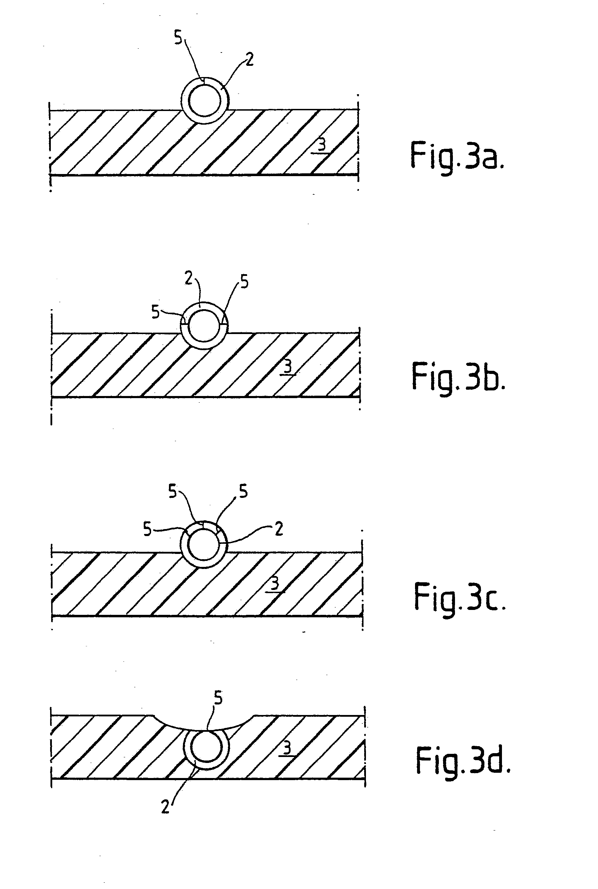

Referring to the drawings, Fig. 1 shows an aqueous humour drainage device 1 having a drainage tube 2 and a drainage body 3. The tube 2 is made of medical grade silicone rubber, and has a length of 24 mm, a wall thickness of 0.175 mm and a bore of 0.3 mm. The drainage body 3 is a band of medical grade silicone rubber having a width of 9 mm and a thickness of 0.75 mm. The tube 2 is fixed to the band 3 by fusing or gluing (as indicated by the reference numerals 4). In practice, the raised portions of the fusing or glue are removed to leave a flush surface. The tube 2 is formed with one or more slits 5 (see Figs. 3a to 3d) in that portion which is fixed to the band 3. The slit(s) in the tube 2 form a pressure gradient limiting valve. The tube 2 is preformed to follow the arc of a circle of diameter 30 mm.

Fig. 2 shows the device 1 implanted in an eye 10. The eye 10 is an average-sized diseased eye, that is to say one having a diameter of about 25 mm. The curvature of the tube 2 is chosen to match this eye diameter, as will be described below. Similarly, the circumference of the band 3 is chosen so that it is just the right length to be sutured to the sclera 11 of the eye 10 at an equatorial position. In use, the two ends of the band 3 are sutured together after the band has been positioned, and, if necessary, trimmed (that is to say, if it is too long for the eye). Fig. 2 shows the lens 12, the cornea 13, the iris 14 and the anterior chamber 15 of the eye 10. This figure also shows the free end of the tube 2 entering the anterior chamber 15 through an opening in the cyclodialysis tract 16, the opening being formed surgically. Fig. 2 also shows that the preformed curvature of the tube 2 is such that the free end of the tube lies about one third of the distance between the iris 14 and the cornea 13. Moreover, this preformed curvature ensures automatic positioning of the free end of the tube 2 in this manner. This is extremely important, as it is essential that the free end of the tube 2 does not touch the corneal endothelium (the cell area of the back of the cornea 13). If this did happen, the cornea 13 would become opaque, and this is clearly undesirable. By curving the tube 2 in this manner, the free end of the tube 2 is automatically positioned well behind the corneal endothelium, and so this undesirable prospect is prevented without the surgeon having to take special precautions. Moreover, the curvature chose is suitable for automatic positioning well clear of the corneal endothelium in a wide variety of sizes and shapes of human eyes. Obviously, for eyes which are much smaller or larger than the "normal" eye, the curvature of the tube 2 will appropriately be smaller or larger than of 30 mm diameter.

Fig. 3a shows a first form of pressure gradient limiting valve, which is constituted by a single slit 5 in the "top" of the tube 2, the slit 5 having a length of 3 mm. Figs. 3b and 3c show valve modifications having two and three slits 5 respectively. Fig. 3d shows a further modification, in which the tube 2 is fixed (fused) within the body of the band 3, and the slit 5 is formed in the "top" surface of the tube, this top surface being positioned within a concave depression in the band. Depending upon the mechanical properties of the tube wall at the valve site, one or more slits 5 will be required to achieve a desired opening pressure of from 4 to 20 mm of mercury.

The pressure gradient limiting valve is used to control the drainage rate. Thus, the high intraocular pressure which exits immediately after entering the eye tends to drain aqueous humour too quickly. This can lead to large choroidal detachments forming, which in turn are associated with a tendency to damage the lens of the eye. The use of the pressure gradient limiting valve prevents this happening, provided the patient does not adversely affect the eye.

The drainage device 1 described above does not suffer from the disadvantages of prior art devices. In particular, the following points should be noted:—

(a) The surface area of the band 3 is sufficiently large to ensure adequate filtration. The large surface area of the band 3 ensures that there is an adequate flow of aqueous humour at a physiological pressure gradient, that is to say without an excessively high (greater than 18 mm mercury) intra-ocular pressure, even if there is a thick growth of scar tissue. In this connection, it should be appreciated that the inevitable scar formation is (provided it is not excessively thick) advantageous. This is because the scar tissue slows down (filters) the flow rate of aqueous humour to a rate commensurate with that which can be secreted by the eye and taken up by the blood and lymphatic vessels at a physiological pressure gradient.

(b) The tube 2 is firmly fixed to the band 3, so there is no possibility of scar tissue forming around a loose end thereof and blocking the flow of aqueous humour.

(c) The pressure gradient limiting valve not only prevents initial excessive loss of aqueous humour from the anterior chamber 15, but it is also positioned on a drainage body (the band 3) of a large surface area. Consequently, there is no danger of the valve being blocked by scar tissue, and aqueous humour can flow continuously and directly onto the band, from where it can be filtered through scar tissue to the blood and lymphatic vessels.

It will be apparent that the drainage device 1 described above could be modified in a number of ways. For example, the width of the band 3 may lie within the range of from about 5 mm to 15 mm for a "normal" eye, though the preferred range (for the normal eye) is 9 to 12 mm. In this connection, it should be noted that band widths of between 5 and 9 mm would probably only be satisfactory for elderly patients, whose scarring tendency is less than that of younger people. If band widths greater than about 15 mm are used, there may be problems with the rotation of the eyeball within its orbit, and this could lead to double vision. Accordingly, if the band 3 is placed equatorially and symmetrically, a width of 15 mm should be not be exceeded. However, if the band is positioned slightly asymmetrically, a band width of up to about 18 mm for an average eye may be possible. Moreover, the tube 2 and the band 3 need not be made of silicone rubber (though this is currently much the preferred material). These members could, for example, be made of a hydrogel such as hydroxyethylmethacrylate.

It would also be possible to use a preformed angled tube instead of the curved tube. For example, the tube could be angled in and then out so as to follow the shown angled route into the anterior chamber of the eye through the cyclodialysis tract. Although such an angled tube could be used in some circumstances, the curved tube is preferable as inserting a curved tube gives rise to considerably less surgical difficulties. It would also be possible, in some circumstances, to use a straight tube.

Another possible modification would be to form the pressure gradient limiting valve in other ways. For example, if the tube has a wall thickness much less than that mentioned above (0.175 mm), it would be preferable to join the tube to the band, to punch a hole in the band at the junction, to cover both sides of the punched hole with thin membranes, and to form the valve in one of the membranes by making one or more slits therein. The thin membranes could be made of the same material (preferably silicone rubber) as the tube and the band.

As an alternative to fixing or bonding the tube to the band, it would also be possible to fix the tube firmly to the band by a friction fit sleeve made of silicone rubber, so that the tube could not separate from the band (which could lead to blockage by scar tissue).

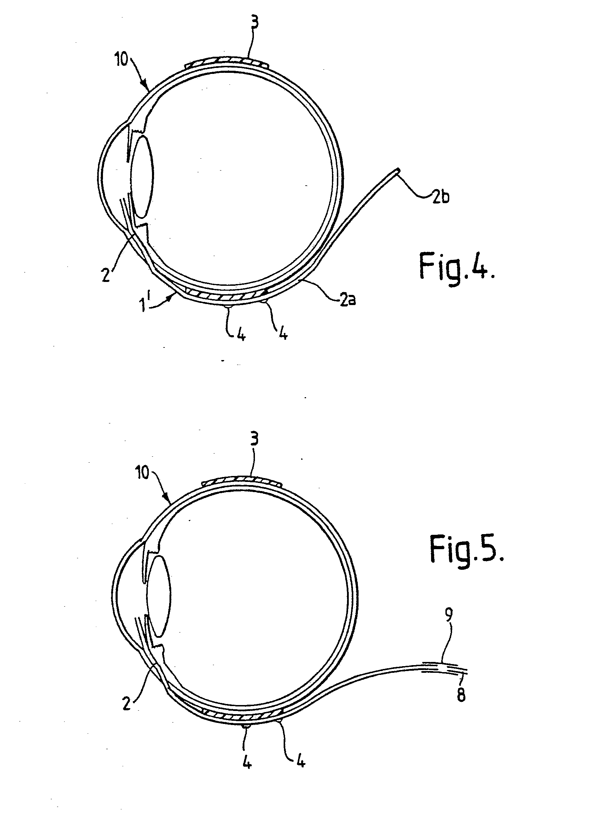

Although it is envisaged that the device 1 described above will be successful in the vast majority of cases (that is to say will ensure that the intra-ocular pressure will be kept below 18 mm of mercury for at least six months), there will inevitably be problems in some cases. In order to deal with such problems, as they arise, without having to make further incisions into the eye, the modified form of device 1' shows in Figs. 4 to 8 could be used. This device 1' is similar to the device 1, in that it has a tube 2 and a band 3. The device 1' is shown implanted in an eye 10. However, the tube 2 has an extension 2a (see Fig. 4) which projects beyond that portion thereof which is fixed to the band 3. The end of the extension 2a is normally blocked by a plug 2b.

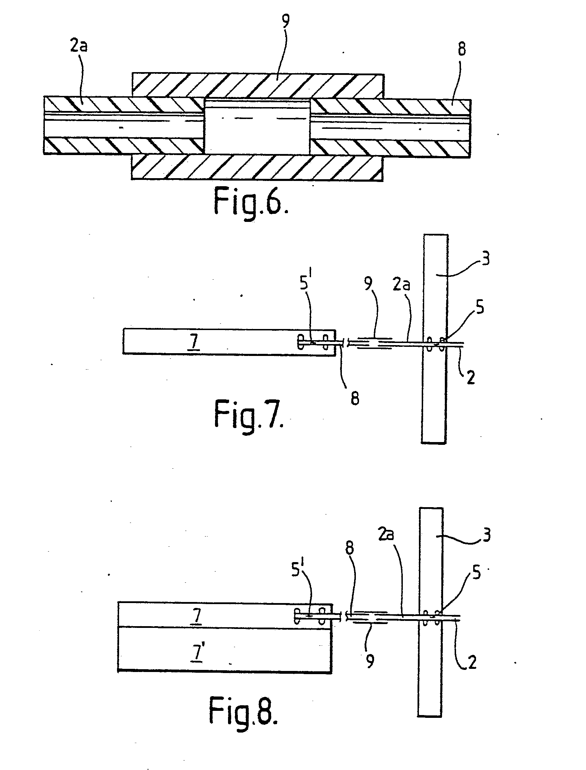

Should the intra-ocular pressure in the eye 10 rise above 18 mm of mercury (owing to a large build up of scar tissue round the band 3, and a subsequent reduction in the rate of filtration), and additional drainage body 7 (see Fig. 7) can be implanted to increase the surface area available for drainage. This drainage body 7 is a strip of medical grade silicone rubber, and it is connected to the tube extension 2a (after removal of the plug 2b) by a silicone rubber tube 8 and a connector 9 (see Fig. 6). This connection is maintained by suturing and by a silicone medical adhesive. The drainage body 7 is inserted in an extra-orbital location, preferably under the scalp of the patient. The tube 8 is provided with a pressure gradient limiting valve 5', this valve being similar to the valve provided in the tube 2—being constituted by one or more slits in the tube 8. The opening pressure of the valve 5' can be chosen to be the same as the valve 5, or it can be greater or less than this valve, the choice being dependent upon the patient concerned. The provision of the additional filtering area thus involves a fairly simple surgical operation, which requires no further surgery in the eye itself.

In the unlikely event of the intra-ocular pressure again rising too high, the filtration area can be further increased by simply adding a further drainage body 7' (see Fig. 8), this further body merely being connected to the drainage body 7 by suturing and by silicone medical adhesive. Here again, this is a simple surgical operation, and involves no further surgery on the eye itself.

In some circumstances, the surface area of the band may prove to be too large, and the rate of filtration may be so large that the intra-ocular pressure approaches zero. In such a case, it would be relatively easy to reduce the surface area of the band by removing a portion thereof, without offending against the conditions as set out in Claim 1.

**Claims**

1. A device for draining aqueous humour from an eye, the device comprising a drainage tube (2) for draining aqueous humour from the anterior chamber (15) of an eye (10), and a drainage body (3) for distributing drained aqueous humour over a relatively large area, the drainage body (3) being sutured, in use, to the sclera of the eye (10), the drainage tube (2) being firmly fixed to the drainage body (3) and opening directly on to a surface of the drainage body (3), the device being provided with a pressure gradient limiting valve (5) having a predetermined opening pressure, characterised in that the drainage body is constituted by a band (3) having a width of at least 5 mm and a length which is sufficient for the band (3) to pass over the sclera of the eye in an equatorial position, the band (3) having a surface area which is at least equal to that of a band of width 5 mm and a length sufficient to pass around the sclera of the eye in an equatorial position, and in that the valve (5) is positioned in the region where the tube (2) is firmly fixed to the band (3).

2. A device as claimed in claim 1, wherein the pressure gradient limiting valve (5) is positioned at that end of the tube (2) which is fixed to the band (3), the valve (5) constituting the opening through which aqueous humour flows directly to said surface of the band (3).

3. A device as claimed in claim 2, wherein the pressure gradient limiting valve is constituted by at least one slit (5) formed in the wall of the tube (2).

4. A device as claimed in any one of claims 1 to 3, wherein the tube (2) is preformed to follow the arc of a circle, the diameter of which is such that, in use, the free end of the tube (2) is positioned within the anterior chamber (15) of the eye (10) closer to the iris (14) than the cornea (13).

5. A device as claimed in claim 4, wherein the diameter of said circle is about 30 mm.

6. A device as claimed in any one of claims 1 to 5, wherein the tube (2) and the band (3) are made of medical grade silicone rubber.

7. A device as claimed in any one of claims 1 to 5, wherein the tube (2) and the band (3) are made of a hydrogel such as hydroxyethylmethacrylate.

8. A device as claimed in any one of claims 1 to 7, wherein the band (3) has a width lying in the range from 9 to 12 mm.

9. A device as claimed in any one of claims 1 to 8, wherein the pressure gradient limiting valve (5) has an opening pressure of from 4 to 20 mm of mercury.

10. A device as claimed in any one of claims 1 to 9, wherein the tube (2) is fused or glued to the band (3).

11. A device as claimed in any one of claims 1 to 10, wherein the tube (2) extends beyond the band (3), the end of the extended portion (2a) being closed.

12. A device as claimed in claim 11, further comprising an additional drainage body (7) and an additional tube (8), one end of the additional tube (8) being connectible to the extension (2a) of the first-mentioned tube (2), after said closed end thereof has been opened, the other end of the additional tube (8) being fixed to the additional drainage body (7) and opening directly on to a surface of the additional drainage body (7).

13. A device as claimed in claim 12, wherein an additional pressure gradient limiting valve (5') is provided in the additional tube (8).

14. A device as claimed in any one of claims 1 to 13, wherein the band (3) is long enough to encircle the eye (10) completely at said equatorial position.

**Patentansprüche**

1. Kammerwasser-Drainagevorrichtung für ein Auge, die eine Drainageleitung (2) zur Kammerwasser-Drainage von der vorderen Kammer (15) eines Auges (10), und einen Drainagekörper (3) zur Verteilung des abgeleiteten Kammerwassers über einen relativ großen Flächenbereich aufweist, wobei der Drainagekörper (3) im Gebrauchszustand an die Sclera des Auges (10) angeheftet ist, die Drainageleitung (2) fest mit dem Drainagekörper (3) verbunden ist und sich direkt auf eine Oberfläche des Drainagekörpers (3) öffnet, und wobei die Vorrichtung mit einem Druckgradientenbegrenzungsventil (5) versehen ist, das einen vorbestimmten Öffnungsdruck hat, dadurch gekennzeichnet, daß der Drainagekörper von einem Band (3) gebildet wird, das eine Breite von wenigstens 5 mm und eine Länge hat, die für das Band (3) so ausreichend bemessen ist, daß es über die Sclera des Auges an einer Äquatorstelle geht, daß das Band (3) einen Oberflächenbereich hat, der wenigstens gleich jenem eines Bandes mit 5 mm Breite und einer Länge ist, die so ausreichend bemessen ist, daß sie um die Sclera des Auges an einer Äquatorialstelle geht, und daß das Ventil (5) in dem Bereich angeordnet ist, an dem die Leitung (2) fest mit dem Band (3) verbunden ist.

2. Vorrichtung nach Anspruch 1, bei der das Druckgradientenbegrenzungsventil (5) am Ende der Leitung (2) angeordnet ist, die fest mit dem Band (3) verbunden ist, wobei das Ventil (5) die Öffnung bildet, durch die das Kammerwasser direkt zur Oberfläche des Bandes (3) fließt.

3. Vorrichtung nach Anspruch 2, bei der das Druckgradientenbegrenzungsventil von wenigstens einem Schlitz (5) gebildet wird, der in der Wand der Leitung (2) ausgebildet ist.

4. Vorrichtung nach einem der Ansprüche 1 bis 3, bei der die Leitung (2) so vorgeformt ist, daß sie einem Kreisbogen folgt, dessen Durchmesser im Gebrauchszustand derart ist, daß das freie Ende der Leitung (2) sich im Innern der vorderen Kammer (15) des Auges (10) und zwar näher an der Iris (14) als an der Cornea (13) befindet.

5. Vorrichtung nach Anspruch 4, bei der der Durchmesser des Kreises etwa 30 mm beträgt.

6. Vorrichtung nach einem der Ansprüche 1 bis 5, bei der die Leitung (2) und das Band (3) aus Silikonkautschuk medizinischer Güte hergestellt wird.

7. Vorrichtung nach einem der Ansprüche 1 bis 5, bei der die Leitung (2) und das Band (3) aus einem Hydrogel, wie Hydroxyethylmethacrylat, hergestellt ist.

8. Vorrichtung nach einem der Ansprüche 1 bis 7, bei der das Band (3) eine Breite hat, die im Bereich von 9 bis 12 mm liegt.

9. Vorrichtung nach einem der Ansprüche 1 bis 8, bei der das Druckgradientenbegrenzungsventil (5) einen Öffnungsdruck von 4 bis 20 mm Quecksilbersäure hat.

10. Vorrichtung nach einem der Ansprüche 1 bis 9, bei der die Leitung (2) an dem Band (3) angeschmolzen oder angeklebt ist.

11. Vorrichtung nach einem der Ansprüche 1 bis 10, bei der die Leitung (2) sich über das Band (3) hinaus erstreckt, wobei das Ende des verlänferten Abschnitts (2a) geschlossen ist.

12. Vorrichtung nach Anspruch 11, die ferner einen zusätzlichen Drainagekörper (7) und eine zusätzliche Leitung (8) aufweist, wobei ein Ende der zusätzlichen Leitung (8) mit der Verlängerung (2a) der erstgenannten Leitung (2) verbindbar ist, nachdem das geschlossene Ende derselben geöffnet worden ist, und wobei das andere Ende der zusätzlich Leitung (8) fest mit dem zusätzlichen Drainagekörper (7) verbunden ist und sich direkt auf eine Oberfläche des zusätzlichen Drainagekörpers (7) öffnet.

13. Vorrichtung nach Anspruch 12, bei der ein zusätzliches Druckgradientenbegrenzungsventil (5') in der zusätzlichen Leitung (8) vorgesehen ist.

14. Vorrichtung nach einem der Ansprüche 1 bis 13, bei der das Band (3) so ausreichend lang ist, daß es das Auge (10) vollständig an der Äquatorialposition umschließt.

**Revendications**

1. Dispositif de drainage d'humeur aqueuse d'un oeil, comprenant un tube d'évacuation (2) pour l'évacuation d'humeur aqueuse de la chambre antérieure (15) de l'oeil (10) et un corps d'évacuation (3) destiné à répartir l'humeur aqueuse évacuée sur une surface relativement grande, le corps d'évacuation (3) étant, lorsque le dispositif est en place, suturé à la sclérotique de l'oeil (10), le tube d'évacuation (2) étant fixé solidement au corps d'évacuation (3) et débouchant directement sur une surface de celui-ci, le dispositif étant pourvu d'une vanne de limitation de gradient de pression (5) ayant une pression déterminée d'ouverture, caractérisé en ce que le corps d'évacuation est constitué d'une bande (3) ayant une largeur d'au moins 5 mm et une longueur suffisante pour passer sur la sclérotique dans une zone équatoriale, cette bande (3) ayant une surface au moins égale à celle d'une bande de 5 mm de large et une longueur suffisante pour passer autour de la sclérotique dans une zone équatoriale, et en ce que la vanne (5) est placée dans la région où le tube (2) est fixé solidement à la bande (3).

2. Dispositif selon la revendication 1, dans lequel la vanne de limitation de gradient de pression (5) est placée à l'extrémité du tube (2) qui est fixée à la bande (3), et constitue l'ouverture par où l'humeur aqueuse va directement à ladite surface de la bande (3).

3. Dispositif selon la revendication 2, dans lequel la vanne de limitation de gradient de pression est constituée d'au moins une fente (5) faite dans la paroi du tube (2).

4. Dispositif selon l'une des revendications 1 à 3, dans lequel le tube (2) est préformé pour suivre un arc de cercle de diamètre tel que lorsque le dispositif est en place, l'extrémité libre du tube (2) soit, dans la chambre antérieure (15) de l'oeil (10), plus près de l'iris (14) que de la cornée (13).

5. Dispositif selon la revendication 4, dans lequel le diamètre dudit cercle est d'environ 30 mm.

6. Dispositif selon l'une des revendications 1 à 5, dans lequel le tube (2) et la bande (3) sont en caoutchouc silicone de qualité médicale.

7. Dispositif selon l'une des revendications 1 à 5, dans lequel le tube (2) et la bande (3) sont constitués d'un hydrogel tel que méthacrylate d'hydroxyéthyle.

8. Dispositif selon l'une des revendications 1 à 7, dans lequel la bande (3) a une largeur comprise entre 9 et 12 mm.

9. Dispositif selon l'une des revendications 1 à 8, dans lequel la vanne de limitation de gradient de pression (5) a une pression d'ouverture de 4 à 20 mm de mercure.

10. Dispositif selon l'une des revendications 1 à 9, dans lequel le tube (2) est uni à la bande (3) par fusion ou collage.

11. Dispositif selon l'une des revendications 1 à 10, dans lequel le tube (2) se prolonge au-delà de la bande (3), l'extrémité du prolongement (2a) étant fermée.

12. Dispositif selon la revendication 11, comprenant en outre un corps supplémentaire d'évacuation (7) et un tube supplémentaire (8), une extrémité de ce tube supplémentaire (8) pouvant être raccordée au prolongement (2a) du tube mentionné en premier (2) après ouverture de ladite extrémité fermée, et l'autre extrémité du tube supplémentaire (8) étant fixée au corps supplémentaire d'évacuation (7) et débouchant directement sur une surface de celui-ci.

13. Dispositif selon la revendication 12, dans lequel une vanne supplémentaire de limitation de gradient de pression (5') est prévue sur le tube supplémentaire (8).

14. Dispositif selon l'une des revendications 1 à 13, dans lequel la bande (3) est suffisamment longue pour entourer complètement l'oeil (10) dans ladite zone équatoriale.

Fig.1.

Fig.2.

Fig.3a.

Fig.3b.

Fig.3c.

Fig.3d.

Fig.4.

Fig.5.

Fig.6.

Fig.7.

Fig.8.